# EUROPEAN PATENT APPLICATION

(11) **EP 1 310 166 A1**
(43) Date of publication of application: **14.05.2003**
(21) Application number: 01936924.8
(22) Date of filing: 08.06.2001
(51) Int. Cl.: A01K 67/027, C12N 5/10

(54) **TRANSGENIC NON-HUMAN MAMMAL AND METHOD OF CONSTRUCTING THE SAME, ANIMAL DISEASE MODEL AND METHOD OF CLARIFYING GENE FUNCTION**

(30) Priority: 16.08.2000 JP 2000247060
(71) Applicant: Kansai Technology Licensing Organization Co., Ltd., Kyoto-shi, Kyoto 600-8815 (JP); Takeda, Junji, Suita-shi, Osaka 565-0871 (JP)
(72) Inventor: TAKEDA, J., c/o OSAKA UNIV. GRADUATE SCHOOL OF MED, Suita-shi, Osaka 565-0871 (JP); HORIE, K., c/o OSAKA UNIV. GRADUATE SCHOOL OF MED., Suita-shi, Osaka 565-0871 (JP)
(74) Representative: Cole, Paul
(86) International application number: JP0104862
(87) International publication number: WO02013602

(57) **Abstract**

The present invention provides a transgenic non-human mammal containing a nonself-contained transposon and/or a transposase gene and a method of producing the same, an animal model of disease, and a method of clarifying gene function.

## Description

### TECHNICAL FIELD

The present invention relates to a transgenic non-human mammal and a method of producing the same, an animal model of disease, and a method of clarifying gene function, which are useful for clarification of gene function.

### BACKGROUND ART

Currently, genome analysis of various organisms including humans is proceeding vigorously worldwide. It is expected that if genome analysis is completed in various organisms, the results of the genomic analysis can be applied to biotechnology and life science, such as clarification of causes of genetic diseases, development of therapies and pharmaceuticals, clarification of history of evolution, and the like.

However, there is a limit in clarifying gene function only from sequence information. The functions of most genes remain unknown. Clarification of the functions of these genes is essential for the effective use of the genes. Clarification of the unknown functions of genes remains to be tackled.

In research for understanding of the functions of genes, a method of specifically disrupting a target gene (Ruvkun et Ausubel; Nature, 289:85-88 (1981)) and the like have been known, in which the homologous recombination property of cells is used to replace a functional allele with an inactivated copy thereof.

Transgenic non-human mammals have been used to clarify gene function. The transgenic non-human mammals are produced by introducing an exogenous gene into a fertilized egg or an early embryo so that the gene is incorporated into an endogenous chromosome of the animal. By deleting, adding, or the like only a particular gene, it is attempted to clarify the relationship between deletion or forced expression of a gene and the phenotype of an individual.

A general technique for producing transgenic mice is described in International Publication WO91-13150 (Ludwig Inst. Cancer Res.). US Patent No. 4,873,191 (Wagner et al.) teaches a mammal having an exogenous DNA, which was obtained by microinjection of the DNA into a mammalian zygote.

Further, a method of efficiently producing mutants of an animal, a plant, or the like has been studied, in which a transposable genetic element (transposon) is inserted or transposed into an endogenous DNA so that the structure of the DNA is changed and the DNA is thus inactivated. Transposons have been available for introduction, addition, and the like of a particular gene into a chromosome.

Transposon as used herein refers to a DNA segment (DNA transposon) capable of moving (transposition) from one site to another on a chromosome. DNA transposons (hereinafter simply referred to as "transposons") are activated by a transposase and are then transposed.

Transposons usually have a repeated sequence (hereinafter referred to as a "transposon sequence") on each end thereof. This repeated sequence is a transposase recognition site. The transposon sequence may include an imperfect repeated portion if the transposon can be transposed by action of a transposase.

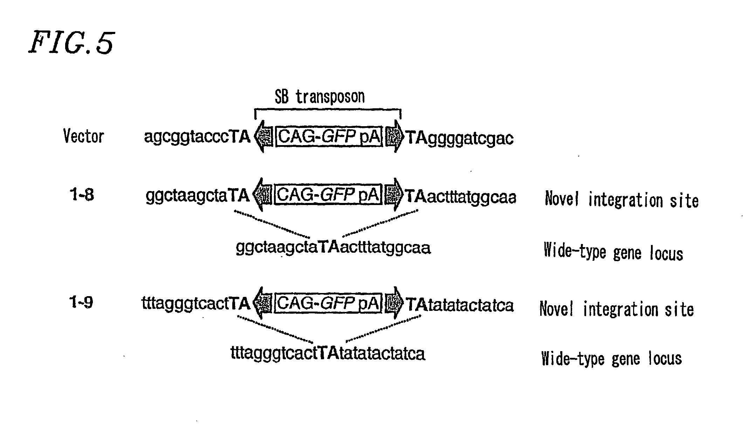

An insertion recognition site having a length specific to transposons within DNA into which a transposon is to be inserted is called a target sequence. For example, in the case of the Sleeping Beauty (SB) transposon system (Z. Ivics et al.; Cell, 91:501-510 (1997)), the target sequence is TA. After a transposon is inserted, the sequence is TA-transposon-TA (see Figure **5**). As target sequences of transposons, for example, TA, ATAT, TATATA, TACA, and the like are known. The literature (Z. Ivics et al.; Cell, 91:501-510 (1997)) relates to expression of the transposon system in cultured cells and does not confirm the effect of the transposon system in a mature mammal individual or organs and the like thereof.

For animal cells, a mariner transposon has been isolated from Drosophila mauritiana, which is a type of fruit fly. This mariner transposon is used to construct a vector. For example, it has been attempted to incorporate a P-element transposon of Drosophila melanogaster into chromosomal DNA of various heterologous organisms. However, the function of the P-element transposon was not maintained due to species specificity. In experiments using flies, such as Muscidae, Sphaeroceridae, Phoridae, or the like, the transposition activity of the P-element was not maintained (Handler et al.; Arch. Insect Biochem. Physiol., 22:373-384 (1993)**)**. In the case of a transgenic zebra fish having an incorporated P-element and reporter gene, genetic expression was not stably obtained (Gibbs et al.; Mol. Mar. Biol. Biotech., 3:317-326 (1994)).

It is known that when Tcl/mariner transposons, which are the most studied eukaryotic transposons, are used in heterologous organisms, transposition is likely to occur since the species specificity of these transposons is relatively low (Z. Ivics et al.; Cell, 91:501-510 (1997)).

An example of a transposon system comprising a transposon, which is reconstructed from such a Tc1/mariner-like transposon, and a transposase, is the above-described Sleeping Beauty (SB) transposon system comprising an SB transposon and an SB transposase. The following examples have been reported: the SB transposon was introduced into human Hela cells and mouse LMTK cells (Z. Ivics et al.; Cell, 91:501-510 (1997)); the SB transposon was introduced into mouse embryonic stem (ES) cells (G. Luo et al.; Proc. Natl. Acad. Sci. USA, 95:10769-10773 (1998)); the activity of a Caenorhabditis elegans-derived Tc1 transposon, which was introduced into human cultured cells, was observed (G. Schouten et al.; Nucleic Acids Res., 26:3013-3017 (1998)). However, for example, in the case of the above-described example in which the SB transposon was introduced into the mouse ES cells, the frequency of transposition of the transposon was 3.5×10⁻⁵ per cell having the introduced exogenous gene at the maximum, which is considerably low. In this case, a large amount of cells had to be used in order to obtain a desired cell. The example that the SB transposon was introduced into the human Hela cells cannot be applied to animal individuals.

For introduction of transposons into mammals, mice having a transposed transposon have been reported, in which the SB transposon and an SB transposase gene were introduced into the genome of somatic cells via the blood (SR Yant et al.; Nature Genetics, 25:35-41 (2000)). In this case, however, the transposition frequency of the transposon was as low as about 5 to 6% of the liver cells having the introduced gene. This method has such a poor efficiency for gene introduction that a transgenic animal line cannot be obtained.

In the case of conventional techniques, it was difficult to randomly introduce mutations into a number of genes of an animal individual *in vivo*. The expression frequency was also low. Therefore, it was necessary to design a general method for mutagenesis of genes.

Therefore, there was a demand for establishment of a technique for solving the above-described problems and introducing a gene into mammals other than a human with high efficiency. It was also desired that such a technique is used to produce transgenic non-human mammals which can be utilized as animal models of human disease for clarification of causes of diseases; studies on therapy, prevention, and the like; and development of drugs, and which are also useful as organ donors.

An object of the present invention is to provide a transgenic non-human mammal and a method of producing the same.

Another object of the present invention is to provide a method of clarifying gene function.

Still another object of the present invention is to provide a method of producing an animal model of disease.

Further another object of the present invention is to identify a gene and a protein involved in disease.

### DISCLOSURE OF THE INVENTION

The present inventors have diligently studied the above-described problems to find a transgenic non-human mammal containing a transposon construct and/or a transposase gene and a method of producing the same, a non-human mammal model of disease, and a method of clarifying gene function. As a result, the present invention was completed.

The present invention relates to items 1 to 28 below.
Item 1. A transgenic non-human mammal, wherein substantially all cells thereof have at least one selected from the group consisting of at least one nonself-contained transposon and at least one signature site.
Item 2. A transgenic non-human mammal, wherein substantially all cells thereof have (i) at least one transposase gene and at least one nonself-contained transposon or self-contained transposon and (ii) at least one signature site.
Item 3. A transgenic non-human mammal, having a transposase gene in a state that allows the transposase gene to express a transposase.
Item 4. A transgenic non-human mammal according to item 1 or 2, wherein the nonself-contained transposon has at least one selected from the group consisting of at least one marker gene and at least one gene expression regulatory sequence.
Item 5. A transgenic non-human mammal according to item 4, wherein the marker gene is a green fluorescence protein (GFP) gene, a yellow fluorescence protein (YFP) gene, a red fluorescence protein (RFP) gene, a blue fluorescence protein (BFP) gene, a cyan fluorescence protein (CFP) gene, a lacZ gene, a luciferase gene, or a Chloramphenicol Acetyl Transferase (CAT) gene.
Item 6. A transgenic non-human mammal according to item 4, wherein the gene expression regulatory sequence is at least one selected from the group consisting of promoters, enhancers, insulators, silencers, splice acceptor sites, and splice donor sites.
Item 7. A transgenic non-human mammal according to item 2, wherein the total number of the signature sites is 0.1% or more, preferably 1% or more, and more preferably 10% or more of the total number of the cells in at least one tissue.
Item 8. A transgenic non-human mammal according to any one of items 1 to 3, wherein the non-human mammal is a mouse or a rat.
Item 9. Amethod of producing a transgenic non-human mammal, comprising introducing a nonself-contained transposon into an animal stem cell or a fertilized egg, and obtaining a nonself-contained transposon-containing non-human mammal from the animal stem cell or the fertilized egg.
Item 10. A method of producing a transgenic non-human mammal, comprising introducing a transposase gene into an animal stem cell or a fertilized egg, and obtaining a transposase gene-containing non-human mammal from the animal stem cell or the fertilized egg.
Item 11. A method of producing a transgenic non-human mammal having a nonself-contained transposon and a transposase gene, comprising crossbreeding a transgenic non-human mammal containing the nonself-contained transposon and a transgenic non-human mammal containing the transposase gene.
Item 12. A transgenic non-human mammal according to item 2, the transgenic non-human mammal can be obtained by a method according to item 11.
Item 13. A method of producing a transgenic non-human mammal having a nonself-contained transposon or a signature site and having fixed transposition, comprising crossbreeding a transgenic non-human mammal having the nonself-contained transposon and the transposase gene in a state that allows the nonself-contained transposon to be transposed, and a non-human mammal containing no transposase gene.
Item 14. A transgenic non-human mammal according to item 1, the transgenic non-human mammal can be obtained by a method according to item 13.
Item 15. A method of producing a transgenic non-human mammal having a nonself-contained transposon and a transposase gene, comprising crossbreeding
   (1) a nonself-contained transposon-containing non-human mammal obtained by introducing a nonself-contained transposon into an animal stem cell or a fertilized egg, and
   (2) a transposase gene-containing non-human mammal obtained by introducing a transposase gene into an animal stem cell or a fertilized egg.
Item 16. A transgenic non-human mammal according to item 2, the transgenic non-human mammal can be obtained by a method according to item 15.
Item 17. A transgenic non-human mammal according to item 16, wherein the marker gene is a green fluorescence protein (GFP) gene, a yellow fluorescence protein (YFP) gene, a red fluorescence protein (RFP) gene, a blue fluorescence gene (BFP) gene, a cyan fluorescence protein (CFP) gene, a lacZ gene, a luciferase gene, or a Chloramphenicol Acetyl Transferase (CAT) gene.
Item 18. A transgenic non-human mammal according to item 16, wherein the gene expression regulatory sequence is at least one selected from the group consisting of promoters, enhancers, insulators, silencers, splice acceptor sites, and splice donor sites.
Item 19. A transgenic non-human mammal according to item 16, wherein the total number of the signature sites is 0.1% or more, preferably 1% or more, and more preferably 10% or more of the total number of the cells in at least one tissue.
Item 20. A transgenic non-human mammal according to item 16, wherein the non-human mammal is a mouse or a rat.
Item 21. A method of producing a transgenic non-human mammal having a nonself-contained transposon or a signature site and having fixed transposition, comprising
   A) crossbreeding
      (1) a nonself-contained transposon-containing non-human mammal obtained by introducing the nonself-contained transposon into an animal stem cell or a fertilized egg, and
      (2) a transposase gene-containing non-human mammal obtained by introducing a transposase gene into an animal stem cell or a fertilized egg, and
   B) crossbreeding the transgenic non-human mammal obtained in step A) having the nonself-contained transposon and the transposase gene in a state that allows the nonself-contained transposon to be transposed, and a non-human mammal containing no transposase gene.
Item 22. A transgenic non-human mammal according to item 1, the transgenic non-human mammal can be obtained by a method according to item 21.
Item 23. A transgenic non-human mammal according to item 22, wherein the marker gene is a green fluorescence protein (GFP) gene, a yellow fluorescence protein (YFP) gene, a red fluorescence protein (RFP) gene, a blue fluorescence gene (BFP) gene, a cyan fluorescence protein (CFP) gene, a lacZ gene, a luciferase gene, or a Chloramphenicol Acetyl Transferase (CAT) gene.
Item 24. A transgenic non-human mammal according to item 22, wherein the gene expression regulatory sequence is at least one selected from the group consisting of promoters , enhancers, insulators, silencers, splice acceptor sites, and splice donor sites.
Item 25. A transgenic non-human mammal according to item 21, wherein the non-human mammal is a mouse or a rat.
Item 26. A method of producing a non-human mammal model of disease, comprising selecting a non-human mammal having a disease or a pathological condition by investigating a phenotype associated with a disease of a transgenic non-human mammal according to item 14.
Item 27. A method of clarifying gene function, comprising investigating a change in a phenotype, and an insertion site and/or a signature site of the nonself-contained transposon or the self-contained non-human mammal, of a transgenic non-human mammal according to any one of items 1, 2, 4-8, 12, 14, 16-20, 22-25 and 28.
Item 28. A transgenic non-human mammal having a changed phenotype, wherein means for adjusting the efficiency of expression of a Bloom gene and a transposon system are combined, and a mutation is introduced into both alleles.

### DEFINITION

As used in the specification and the brief description of the drawings, "GFP" refers to a Green Fluorescence Protein. Its corresponding gene is referred to as a "GFP gene". In the drawings, "GFP" refers to the GFP gene.

As used in the specification and the brief description of the drawings, transposons and transposases in the Sleeping Beauty (SB) transposon system are referred to as SB transposons and SB transposases, respectively. In the drawings, "SB" refers to a Sleeping Beauty transposase gene.

### ELEMENTS USED IN THE INVENTION

In the present invention, examples of non-human mammal animals, which can be used to produce animal models of disease and/or clarify gene function, include swine, monkeys, cattle, horses, goats, sheep, cats, dogs, rabbits, mice, rats, hamsters, and the like, and more preferably, mice or rats. Here, the non-human mammals of the present invention include not only non-human mammal individuals but also a portion of the individuals and organs of the individuals . These animals are useful as models of human disease or donors for organ transplantation.

Various DNA sequences (e.g., marker genes, gene expression regulatory sequences, desired genes, and the like) can be inserted into a portion interposed between transposon sequences. Various elements can be optionally combined with a transposon sequence to construct a transposon construct.

In the present invention, any cell having a potential to differentiate into a non-human mammal individual can be used as a cell into which a transposon construct or a transposase gene is introduced. Examples of such a cell include animal stem cells and fertilized eggs. As used herein, the term "animal stem cell" includes the above-described cells capable of becoming a non-human mammal individual. Preferably, ES cells may be utilized. Unfertilized eggs having a transferred nucleus can be used as fertilized eggs. The ES cells used herein include one commercially available from LifeTech Oriental. For example, it is known that an unfertilized egg having a transferred nucleus was obtained by introducing a nucleus derived from a mature mouse somatic cell into an enucleated oocyte, and such an egg underwent differentiation (T. Wakayama et al.; Nature, 394:369-374 (1998)).

The transgenic non-human mammal of the present invention includes not only a founder (primary generation) containing one or both of a transposon construct and a transposase but also a transgenic non-human mammal line established based on the founder. Further, the present invention encompasses organs, tissue, egg, sperm, and fertilized egg of the transgenic non-human mammal line; cell lines established from the transgenic non-human mammal line; and transgenic non-human mammal clone individuals produced from the transgenic non-human mammal line.

The transposon construct of the present invention is constructed by combining various elements with a transposon sequence, and can be introduced into animal stem cells, fertilized cells, or the like.

In the present invention, "transposon sequence" refers to a native or artificial transposon DNA sequence which is recognized by a transposase and is transposable within transgenic non-human mammal cells.

In the present invention, any transposon sequence and transposase gene can be used for transgenic non-human mammals no matter whether it is endogenous or exogenous. Preferably, exogenous transposon sequences and transposase genes can be used.

A DNA transposon is used as a transposon of the present invention.

Transposons include self-contained transposons which encode, within themselves, an active enzyme transposase capable of catalyzing their transposition and nonself-contained transposons which lack transposase activity. A nonself-contained transposon is used when transgenic non-human mammals having a transposon sequence or a transposase gene are crossbred to obtain a transgenic non-human mammal having the transposon sequence and the transposon gene or when an attempt is made to obtain a transgenic non-human mammal having a transposon sequence having fixed transposition (having no transposase gene). Both self-contained and nonself-contained transposons can be used when an attempt is made to obtain a transgenic non-human mammal containing both a transposon and a transposase or a transgenic non-human mammal containing a signature site but no transposon or transposase gene.

Self-contained transposons can be converted to nonself-contained transposons by using a Cre/loxP system described below. Specifically, a transgenic non-human mammal having a loxP sequence on the opposite ends of a transposase gene is crossbred with a transgenic non-human mammal having Cre to cut off the transposase.

Further, transposons include host-dependent and host-independent transposons, either of which may be used. In general, host-independent transposons may be used.

In the present invention, a transposon system comprising a transposon and a transposase gene is used after only the transposon and/or only the transposase gene are excised from the system or the system is converted to a nonself-contained transposon by inactivating the transposase. A transposon system useful in implementation of the present invention comprises any sequence transposable within transgenic non-human mammal cells. Preferably, members of the Tc1/mariner superfamily may be used as the system. Examples of the Tc1/mariner superfamily members include Tc1 transposon families, such as Tc1, SB, Minos, Txr, Tc3, and the like; mariner transposon families, such as Caenorhabditis elegans, Mos1, Hyalophora cecropia, and the like; and Pogo transposon families, such as Pogo, Tigger, Tc4, and the like (RH Plasterk et al.; Trends in genetics, 15: No. 8: 326-332 (1999)). Most preferably, SB (Sleeping Beauty) transposon may be used. A nonself-contained transposon can be obtained by removing or inactivating the transposase gene of a self-contained transposon.

As to how to obtain general transposon systems, for example, see literature, such as Z. Ivics et al.; Cell, 91:501-510 (1997) for an SB transposon system, A. G. Klinakis etal.; EMBO Reports 1,5:416-421 (2000) for a Minos transposon, and the like.

Hereinafter, examples in which nonself-contained transposons were used will be described. Instead of nonself-contained transposons and transposases, self-contained transposons containing a transposase gene can be used.

In addition to a transposon sequence, a transposon construct used in the present invention may comprise at least one of: a sequence (pBluescript or the like) derived from a vector used in construction of the transposon construct; at least one type of marker gene (including a reporter gene and a selectable marker gene) ; a gene expression regulatory sequence, such as a signal sequence (including a nuclear translocation signal, a membrane binding signal, and the like) , a promoter, an enhancer, a silencer, an insulator, a splice acceptor, a splice donor, loxP, FRT, a poly A signal, a transcription termination signal, and the like. The present invention is not so limited. A transposon construct of the present invention may comprise only a transposon sequence. A transposon construct of the present invention may be a self-contained transposon containing a transposase gene.

Transposon constructs do not necessarily comprise any component other than a transposon sequence. However, it is preferable that transposon constructs comprise a reporter gene such as GFP, yellow fluorescence protein (YFP), or the like and a selectable marker gene such as an antibiotic resistance gene, where a promoter is linked to the 5' end of each gene and a poly A sequence is linked to the 3' end of each gene.

For example, when a transposon sequence is inserted into an exon portion of a gene as a result of transposition thereof or when a transposon sequence has an inserted gene expression regulatory sequence, such as a promoter, an enhancer, a silencer, a splice acceptor site, a splice donor site, or the like, the function of a gene at the insertion site of the transposition is disrupted, lowered, or activated.

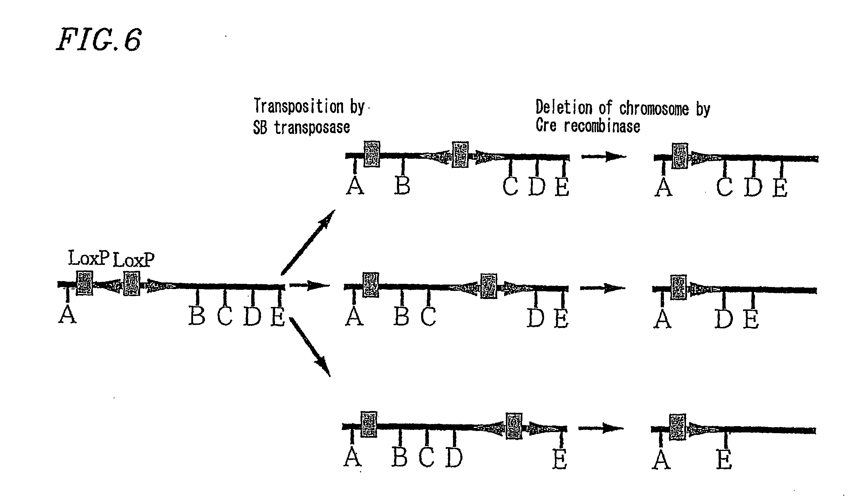

Transposon sequences are mostly transposed on the same chromosome. For this reason, in transposon constructs, a loxP sequence is provided inside and outside a transposon sequence so that the transposon sequence is transposed by a transposase. Next, a Cre recombinase is used to delete a chromosomal region between the loxP sequence outside the transposon sequence and the loxP sequence inside the transposon sequence. As a result, this region has only one member of a pair of genes which are usually present in the region. If additional mutations are introduced, it is easy to perform screening for recessive inheritance (see Figure **6**).

A transposase gene of the present invention can be introduced into cells, such as fertilized eggs and the like, via a transposase construct comprising the transposase gene and other various components. In the present invention, "transposase gene" refers to a gene from which a transposase having transposon transposing activity is expressed. It is preferable that the gene is introduced into animal stem cells , fertilized eggs, or the like in a state that allows the gene to express a transposase.

Components of transposase constructs which can be used in the present invention, include, are not limited to, for example, promoters, enhancers, poly A signals, and the like, in addition to transposase genes. A promoter is preferably incorporated into the 5' end of a transposase gene. Alternatively, a promoter may be present at the 3' end of a transposase gene. Other promoters are not essential. In the present invention, a preferable transposase construct comprises a promoter, a transposase gene, and a poly A in this order from the 5' end.

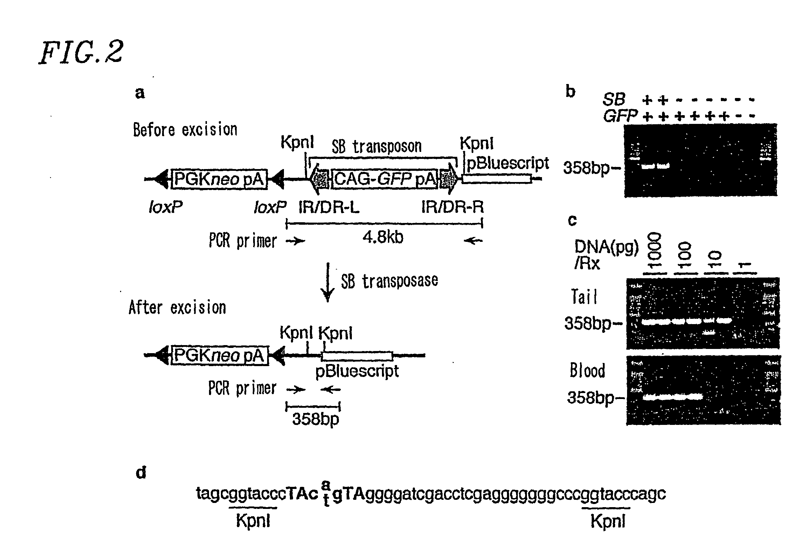

In the present invention, "signature site" refers to a site which emerges as a result of excision and transposition of a transposon. For example, when an SB transposon is used in the present invention, a signature site comprises a sequence "TAcagTA" or "TActgTA" where three bases of the terminus sequence of the transposon is inserted into a TA repeat of a target sequence (Figure **2d**). Note that even when a transposon is moved, a resultant signature site may not have the above-described perfect specific sequence. In the present invention, a site having such an imperfect sequence is regarded as a signature site.

In item 28, "introducing a mutation into both alleles" indicates both that this is done when a transposon is excised from a genome and is transposed to a different portion on the genome and that this is done by a signature site.

Examples of a selectable marker gene or a reporter gene included in a marker gene used in the present invention, include: a Neomycin resistance gene, a Puromycin resistance gene and a Hygromycin resistance gene which confer antibiotic resistance; a green fluorescence protein (GFP) gene; a yellow fluorescence protein (YFP) gene; a red fluorescence protein (RFP) gene; a blue fluorescence gene (BFP) gene; a cyan fluorescence protein (CFP) gene; a lacZ gene; a luciferase gene; a Chloramphenicol Acetyl Transferase (CAT) gene; and the like, which may be appropriately selected according to the purpose and necessity.

In the present invention, the term "promoter" refers to a DNA region which is located upstream of a transcription initiation site and is involved in recognition and binding of a RNA polymerase and other proteins for initiating transcription.

Promoters which may be used in the present invention are not particularly limited as long as they are active in cells or tissue of non-human mammals. Examples of such promoters include a PGK promoter, a CAG promoter, and a PolII promoter.

The basic transcription activity of a promoter can be increased in the presence of an enhancer sequence. Although the underlying mechanism has not been clarified, it is known to those skilled in the art that a certain type of enhancer regulatory sequence already clarified increases the transcription rate of a promoter when it exists near the promoter.

Enhancers which may be used in the present invention are not particularly limited as long as they are active in cells or tissue of non-human mammals. Examples of such enhancers include a human cytomegalovirus (HCMV) enhancer, and the like, which may be appropriately selected according to the purpose and necessity.

When a promoter or an enhancer having expression inducibility depending on a hormone or an antibiotic, such as tetracycline, is used in a transposase construct, transposition of a transposon can be regulated in a time-specific manner or a tissue-specific manner with or without administration of the hormone or the antibiotic, such as tetracycline.

Silencers which may be used in the present invention are not particularly limited as long as they are active in cells or tissue of non-human mammals. In one embodiment of the present invention, a silencer may be used as a component within a transposon sequence to inhibit expression of mutation. An exemplary silencer is described in S. Sawada et al.; Cell, 77:917-929 (1994)) and the like.

Insulators which may be used in the present invention are not particularly limited as long as they are highly active in cells or tissue of non-human mammals. In one embodiment of the present invention, an insulator can be used as a component within a transposon sequence to allow expression within the transposon sequence without depending on sequences surrounding the insertion site of the transposon sequence, or the like. An exemplary silencer is described in JH. Chung et al.; Cell, 74:505-514 (1993)) and the like.

According to one embodiment of the present invention, a transposon sequence containing a transcription termination sequence may be introduced into non-human mammals. Examples of such a transcription termination sequence include C2 termination signal (R. Ashfield et al.; EMBO J., 10:4197-4207 (1991)).

Signal sequences which may be used in the present invention are not particularly limited as long as they are highly active in target cells or tissue. In one embodiment of the present invention, by using a signal sequence (e.g., a transmembrane signal) as a component of a transposon construct, it is possible to gather markers (e.g., GFP) at a target site (e.g., a membrane surface) in cells having a modified gene function, thereby increasing the efficiency of analysis of gene function.

According to one embodiment of the present invention, a transposon sequence containing a splice acceptor site having strong or weak activity may be introduced into non-human mammals. As such a splice acceptor site, a nucleotide sequence containing a sequence AG, which is located at the 3' terminus of an intron which works in RNA splicing, is illustrated, for example.

According to one embodiment of the present invention, a transposon sequence containing a splice donor site having strong or weak activity may be introduced into non-human mammals. As such a splice donor site, a nucleotide sequence containing a sequence GT, which is located at the 5' terminus of an intron which works in RNA splicing, is illustrated, for example. The strength of a splice acceptor and a splice donor can be evaluated according to the length of an intron sequence capable of being spliced, for example.

Transposons are randomly transposed. In one embodiment of the present invention, the frequency of transpositions may be adjusted by providing promoters or enhancers having various levels of expression inducibility within a transposase construct in order to prevent a fatal mutation caused by transposition of a transposon, such as severe growth failure occurring in cells. Thereby, it is possible to clarify the function of a gene essential for survival.

By adjusting the strength of the activity of a gene expression regulatory sequence (a splice acceptor, a splice donor, an enhancer, or the like) in a transposon construct, the level of disruption of gene function may be adjusted.

In order to modify a gene more flexibly and precisely, a gene trap vector comprising a splice acceptor, a splice donor, a poly A addition signal, and a promoter as essential components, may be used as a transposon construct in the present application.

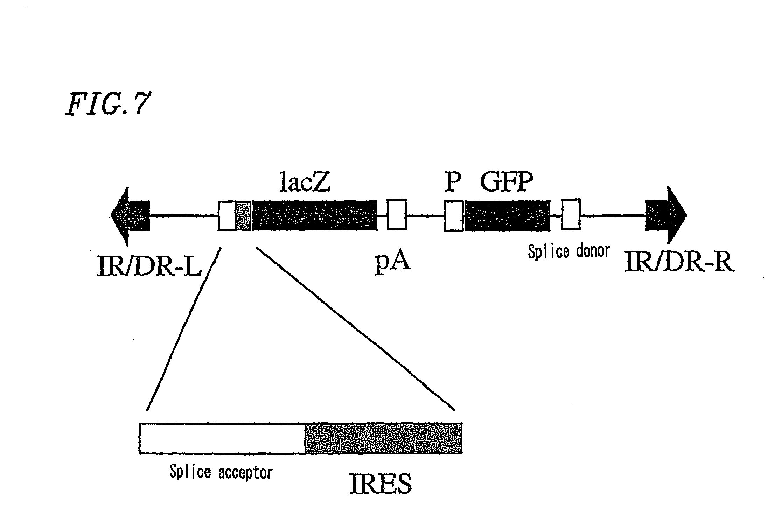

As an example, the trap vector comprises a splice acceptor, an IRES (international ribosome entry site) sequence, a lacZ marker, a poly A addition signal, a CAG promoter, a GFP marker, and a splice donor in this order from the upstream, between transposon sequences (see Figure **7**). Here, the splice acceptor and the splice donor are used to catch an endogenous gene. The IRES sequence causes the lacZ gene to be expressed independently from endogenous genes. Instead of the above-described components, other components having a similar function may be used in the trap vector. In this vector, the GFP gene is controlled by a ubiquitous and strong CAG promoter. In addition, the splice donor is located downstream of the GFP gene. Therefore, fluorescence can be used to detect when a gene is trapped. At the same time, the trapped gene can be identified by performing 3' RACE using RNA at the 3' terminus. Since the splice acceptor is concurrently present, the expression site of the trapped gene can be specified by analyzing expression of the lacZ gene located downstream of the splice acceptor.

In the present invention, a known method can be used to introduce a transposon sequence or a transposase gene into cells (ES cells, fertilized eggs, or the like) capable of becoming non-human mammals. Preferably, examples of an introduction method for ES cells include retrovirus vectors, electroporation, and the like; and examples of an introduction method for fertilized eggs include microinjection and the like. As a basic patent relating to production of transgenic animals using the microinjection method, US Patent No. 4,873,191 (Wagner et al.) is illustrated.

The ES cell having the introduced transposon sequence or transposase gene is injected into a blastocyte of a non-human mammal which is in turn transplanted into the uterus of a pseudopregnant female non-human mammal. For example, if the non-human mammal is a mouse or a rat, a chimera mouse is born two weeks after the transplant. When a fertilized egg is used, a transposon sequence or a transposase gene is injected into the male pronucleus of the fertilized egg and the resultant fertilized egg is transplanted into the oviduct of a pseudopregnant female non-human mammal. For example, when the non-human mammal is a mouse, a founder mouse is born about 20 days after the transplant.

### FEATURES OF THE METHOD OF THE PRESENT INVENTION

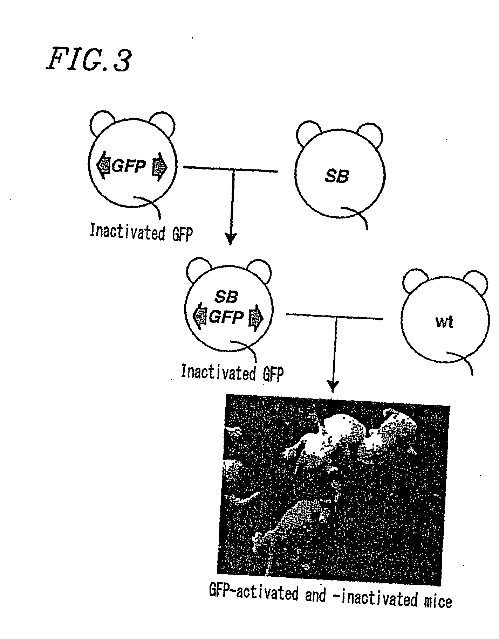

In a method according to one embodiment of the present invention as illustrated in Figure **3**, a "transgenic non-human mammal having a transposase gene" and a "transgenic non-human mammal containing a nonself-contained transposon" can be crossbred to obtain a "non-human mammal containing the transposase gene and the transposon". With this method, similar parents can be crossbred to obtain child mammals having an identical gene. This method makes it possible to know in advance the influence of introduction of only a transposon construct into a mammal on a phenotype of the mammal. Similarly, the method makes it possible to know in advance the influence of introduction of only a transposase construct into a mammal on a phenotype of the mammal.

Alternatively, it is possible to obtain a "transgenic non-human mammal containing a trasnposase gene and a transposon" into which the trasnposase gene and the transposon are initially introduced without crossbreeding. This method does not require crossbreeding of parents, resulting in good efficiency in terms of labor, time, and cost.

In this "transgenic non-human mammal containing a trasnposase gene and a transposon", since the transposon is contained in a state that allows the transposon to be transposed, the transposon can be transposed on a chromosome . This transposition can disrupt, lower, or activate gene function at any site on the chromosome.

Further, a "transgenic non-human mammal containing a trasnposase gene and a transposon" arid a "non-human mammal containing no transposase" can be crossbred to obtain a "non-human mammal having a transposon but no transposase gene" . If a transposase is interposed by loxPs, a non-human mammal containing Cre may be used for crossbreeding.

Referring to Figure **3** as one embodiment, a "transgenic non-human mammal, in which substantially all cells thereof contain (i) at least one transposase gene and at least one nonself-contained transposon or self-contained transposon and (ii) at least one signature site" (item 2 of the present invention) corresponds to a "transgenic non-human mammal having both a transposon sequence (TP) containing GFP as an arbitrary component and a transposase gene (SB) (hereinafter also referred to as a "TP-SB mammal")". This transgenic non-human mammal is obtained by crossbreeding a "non-human mammal having a transposon sequence (TP) containing GFP as an arbitrary component but no transposase gene (SB) (hereinafter also referred to as a "TP mammal")" and a "non-human mammal having a transposase gene (SB) but no transposon sequence (hereinafter also referred to as an "SB mammal")".

The non-human mammal of item 2 is induced from an animal stem cell or a fertilized egg having a transposon or a signature site. Therefore, essentially all cells contain a transposase gene. In fact, a transposon may not leave a signature site when the transposon is excised and a transposon may not be transposed. Therefore, item 2 describes "substantially all cells". "Substantially all cells" is meant all cells except for such a particular cell (s). In each cell of the above-described non-human mammal, a transposon is randomly transposed. For this reason, no uniform mutation is found in a whole individual among genetic mutations introduced by the transposon.

On the other hand, of the transgenic mammals of item 1, a transgenic mammal obtained by crossbreeding a "TP-SB mammal" and a "non-human mammal containing no transposase" is a transgenic mammal in which a genetic mutation based on a signature sequence of the "TP-SB mammal" has already been present since the fertilized egg stage and the common signature site is contained in substantially all cells of the mammal individual. The term "substantially all cells" of item 1 has the same meaning as that of item 2.

In the present invention, a desired transgenic non-human mammal may be obtained by prescreening. As a prescreening method, a genetrap method can be used, for example (Zambrowicz et al.,; Nature, 392:608-611 (1998); Gossler, A. et al.; Science, 244:463-465 (1989); Skarnes, W.C. et al.; Genes Dev, 6:903-918 (1992); and Friedrich, G. et al.; Genes Dev, 5:1513-1523 (1991)).

Thus, prescreening is performed to select in advance transgenic non-human mammals desirable for clarification of gene function. Thereafter, crossbreeding over two or more generations or other appropriate means can be performed to obtain a transgenic non-human mammal in which both genes on a pair of chromosomes are mutated.

A method of analyzing the phenotype of a gene by disrupting the gene is an effective means for clarifying gene function. There are two big problems to be overcome in order to analyze phenotypes by exhaustive gene disruption for a mammal individual, particularly a mouse. The first problem is that there is no satisfactory technique for exhaustively disrupting genes so as to investigate gene function from phenotypes, i.e., so-called forward genetics. The second problem is that since there are a pair of genes (both alleles) , a phenotype does not appear if only one member of the pair of genes is disrupted. Currently, individuals having one disrupted member of a pair of genes are crossbred in order to introduce a mutation into both alleles. In other words, a long time is required for crossbreeding to obtain an individual in which a mutation is introduced into both alleles.

The first problem can be overcome by a transposon system newly developed in the present invention. The second problem can be overcome by a method of rapidly introducing a mutation into both alleles (hereinafter referred to as a "double allele mutation introduction method").

As a specific method for overcoming the second problem, a Bloom gene knockout mouse, in which cells having a mutation in both alleles frequently appear, can be used (G. Luo et al.; Nature Genetics, 26:424-429 (2000)). Note that a perfect Bloom gene knockout mouse is fatal (N. Chester et al.; Gene and Dev., 12:3382-3393 (1998)), and therefore, the second problem may not be overcome.

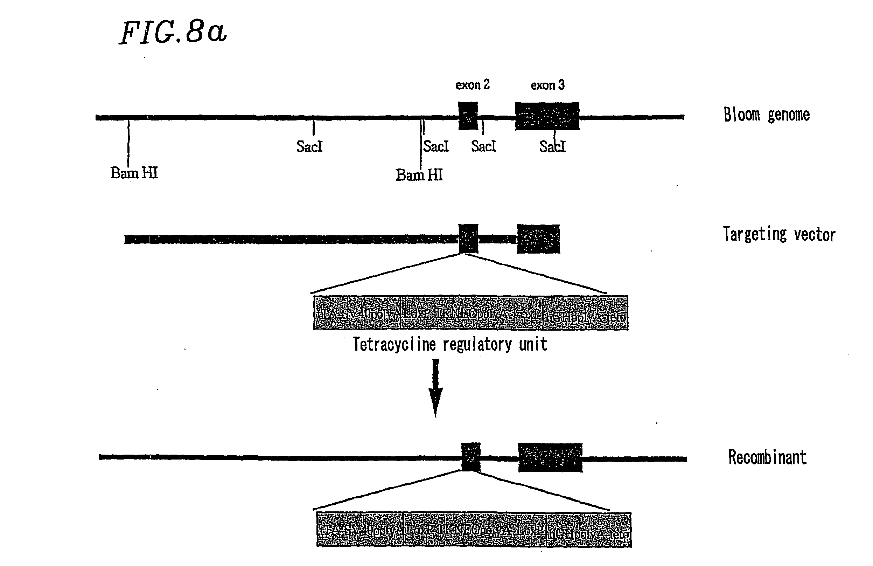

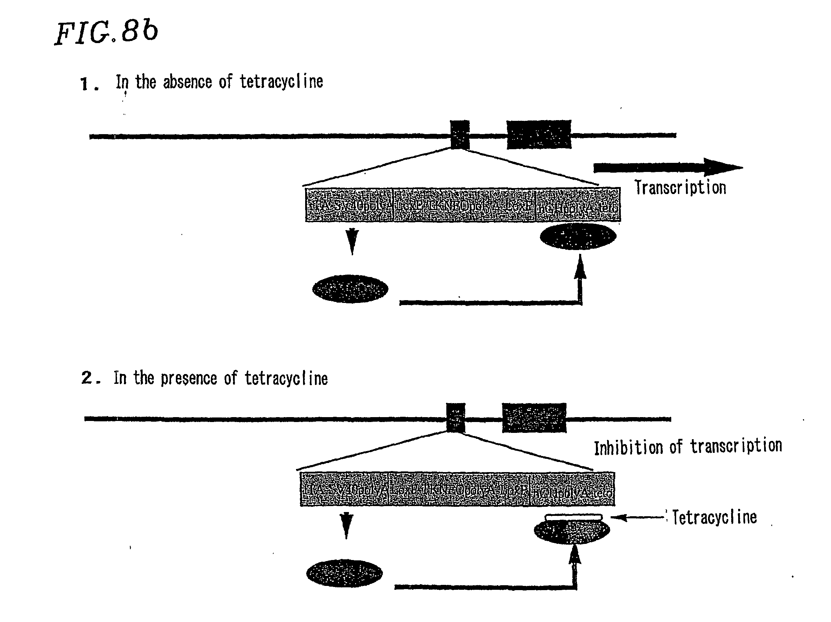

The present inventors are producing a mouse , in which expression of the Bloom gene can be arbitrarily adjusted, using a tetOFF system (CT. Bond et al.; Science, 289:1942-1946 (2000)) (see Figure **8**). The Bloom gene encodes DNA helicase. If the activity of DNA helicase is lost, sister chromatid exchange (SCE) occurs, and at the same time, exchange with another chromatid occurs. Therefore, the lack of the bloom gene causes recombination in tetraploids. In this case, a cell in which both members of a pair of genes are mutated may occur in a part of an individual.

For example, if the Bloom gene is switched ON/OFF in a tetracycline dependent manner (A. Kistner et al.; Proc. Natl. Acad. Sci. USA, 93:10933-10938 (1996)), by adjusting the time of supplying tetracycline or the time of supplying no tetracycline, recombinations can be more frequently induced in a time-specific manner so that a cell having a pair of mutated genes is likely to occur. Therefore, a non-human mammal having a pair of mutated genes can be obtained without repetition of crossbreeding. As a method of introducing mutation in a time-specific manner, for example, pellets are continuously administered perorally into a non-human mammal so that a fetus having a pair of mutated genes can be obtained.

A means for regulatably expressing the Bloom gene (e.g., a tetracycline regulatable unit) is introduced in combination with a transposon system. For example, before crossbreeding, a means for regulatably expressing the Bloom gene is introduced into a fertilized egg or the like, into which a transposon construct, a transposase, a self-contained transposon, or the like is to be introduced. The obtained mouse having an introduced transposon transposition site is treated with a means for inhibiting expression of the Bloom gene (e.g., administration of tetracycline) so that a genetic mutation obtained by a transposon system is introduced into both alleles, thereby making it possible to rapidly determine phenotype.

In the present invention, when no selectable marker gene is used, DNA may be extracted from cells of a non-human mammal and may be then screened by investigating the presence or absence of transposition by southern blotting.

According to the present invention, it is possible to achieve efficient transposition of a transposon sequence in animals *in vivo*. Non-human mammals having various phenotypes can be efficiently and randomly obtained by a method of introducing mutation using a transposon, as compared to other methods . The transgenic non-human mammal of the present invention provides a considerably useful tool for clarifying complex life processes in gene function research since various genetic mutations can be introduced.

The frequency of expression of a transposon in cells is 3.5×10⁻⁵ per cell at maximum, which is considerably low, as described in Proc. Natl. Acad. Sci. USA, vol. 95:10769-10773 (1998). In contrast, according to the present invention, the frequency of transposon expression among individuals, for example, was 42% of all mice and 80% (at maximum) of GFP gene-positive mice in an Example below. These values are significantly high. The present invention is the first to find that the transposition efficiency of a transposon was dramatically increased by the transposon expression system made of an aggregate of cells, such as animals or tissue, organs, and the like thereof.

According to one embodiment of the present invention, it is possible to screen transgenic non-human mammals having an introduced transposon construct for individuals having a randomly introduced mutation using a marker or other means. This is useful as means for clarifying gene function.

For exhaustive analysis of gene function, it is necessary to cause a transposon to be transposed to a greater number of sites on a genome. According to item 2 of the present invention, it is possible to obtain a "transgenic non-human mammal, in which substantially all cells thereof contain (i) at least one transposase gene and at least one nonself-contained transposon or self-contained transposon and (ii) at least one signature site" . For example, according to an example of the present application using mice, a signature site could be introduced at a rate of at least one per 10 cells, resulting in "seed mice" having a variety of cells in a mosaic pattern. Therefore, by producing mutated mice from different seed mice, it is possible to exhaustively introduce a mutation into substantially all genes the number of which is believed to be at least about 30,000. Therefore, in analysis of non-human mammals having mutations, since the present invention can achieve a considerably high level of expression frequency of genetic mutations, a number of functional changes by mutations can be simultaneously analyzed from if a single non-human mammal individual having a plurality of mutations is obtained. Thus, gene function can be highly efficiently clarified.

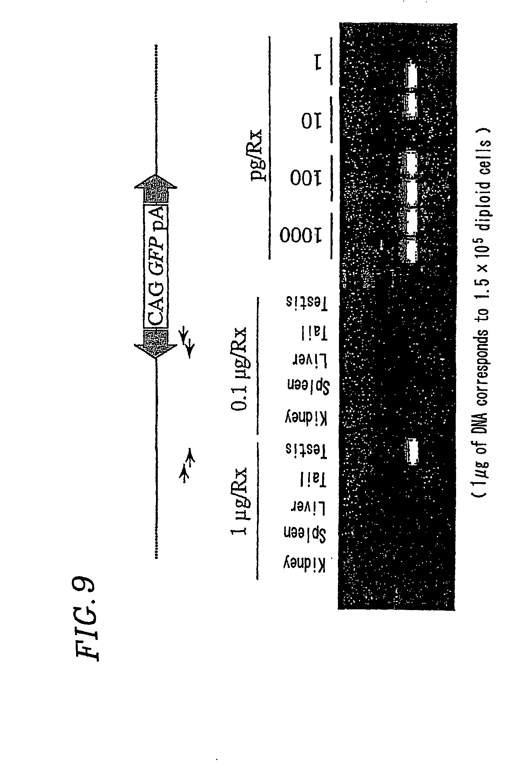

When mice are used for introduction of mutations, for example, only one transgenic mouse is obtained from a single cell by a conventional method of introducing a mutation into an ES cell. On the other hand, according to the transgenic non-human mammal production method of the present invention, when a "transgenic non-human mammal (e.g., a mouse), in which substantially all cells thereof contain at least one selected from the group consisting of at least one nonself-contained transposon and at least one signature site" is used as a seed mouse, it is possible for the single individual to give birth to 100,000 types of transgenic individuals (see Figure **9**). In other words, the present invention has a merit that a single mouse can give birth to offspring having a huge number of types of transpositions. Thus, it is possible to obtain various mutated individuals for clarification of life processes.

According to the present invention, by obtaining and crossbreeding transgenic non-human mammals, it is possible to obtain non-human mammals having fixed transposition which are useful for clarification of gene function. As usedherein, "fixed transposition" means that the number of signature sites produced by transposition of a transposon is not increased due to the lack of an active transposase. Specifically, this indicates either the case where at least one signature site and a transposon are present but no or an inactivated transposase(s) is present or the case where at least one signature site is present but no transposon is present.

If such a transgenic mammal individual is obtained, a type of gene function can be simply analyzed by investigating a corresponding single individual. It is also possible to investigate an individual having a particular mutation about the influence of the mutation on the individual in the course of its growth. Among the above-described non-human mammals, a non-human mammal lacking a certain function can be used to confirm a causative gene involved in a particular function as follows. For example, a transposon sequence containing a splice acceptor is used to add a transposase into a fertilized egg of the non-human mammal or crossbreed the non-human mammal with a non-human mammal having a transposase so as to remove the transposon sequence. It is then determined whether the function can be recovered as a result of the removal of the transposon sequence.

In the present invention, mutations are introduced by transposons. Therefore, mutation introduction sites can be easily detected by an appropriate method, such as PCR or the like, using a signature sequence or a sequence derived from a transposon construct, as compared to when a mutation is introduced using a mutation inducing substance or the like.

In an embodiment of the present invention, by introducing a genetic mutation into a non-human mammal individual but not culture cells, it is possible to analyze gene function in individuals. It is also possible to introduce a genetic mutation into *in vivo* tissue of a non-human mammal individual, which is difficult to handle while the non-human mammal individual remains alive, without external manipulation.

Further, transposition sites differ even within the same tissue, so that there are genetically different cells. Therefore, the lineage of cells, such as proliferation, differentiation, and the like, can be systematically investigated in any tissue and organs, such as the blood system, the immune system, and the like.

According to a second embodiment of the present invention, a novel non-human mammal (particularly, a mouse) of the present invention provides a model system useful for clarification of gene function. This embodiment of the present invention may provide a model system of disease for studies on genetic disease in *in vivo* animal models. In the system, examples of disease genes to be introduced into animal models include human disease causative genes, homologous genes of non-human mammals with the human disease causative genes, full-length cDNA genes, cDNA gene fragments, full-length genomic DNA genes, and genomic DNA gene fragments. Such a disease causative gene is not particularly limited. Any disease causative gene can be used as long as it can be introduced into non-human mammals and the resultant transgenic non-human mammals can be studied as animal models of human disease. Human disease causative genes are preferable.

According to one embodiment of the present invention, when a transposon containing various enhancers is transposed nearproto-oncogenes, cancer is eventually expressed in cells containing these genes. Therefore, it is possible to perform screening for proto-oncogenes. In particular, when a transgenic non-human mammal containing a transposon sequence and a transposase gene is used, cancer undergoes metastasis over the whole body as well as tissue since proto-oncogenes are clonally expressed. At the same time, reduction, disruption, or activation of gene function due to transposition randomly proceed in each animal cell. It is expected that a plurality of cancers occur in the same individual. Therefore, clarification of gene function involved in cancer can be efficiently developed. Further, when a plurality of cancers are confirmed in the same individual, it is possible to investigate whether or not cancerous cells are derived from the same cell by investigating whether or not the insertion site of a transposon vector is the same for the cancerous cells. Thus, the present invention may contribute to research on the mechanism of cancer metastasis.

According to a third embodiment, the transgenic non-human mammal of the present invention may be used as a donor for organ transplantation. Examples of organs which are considered to be used as donors for heterograft to a human, include neurons, heart, lung, liver, pancreas, kidney, cornea, skin, and the like. In this case, as an introduced gene, a gene having a function of possibly reducing rejection or a gene having a function of expectably increasing acceptance are preferable in heterograft, for example.

Hereinafter, a method of producing an expression vector used in the present invention will be described, illustrating a specific example. Note that the method can be easily carried out by those skilled in the art where elements, such as a starting plasmid, a promoter, and the like, used in the specific example are replaced with their equivalents.

### BEST MODE FOR CARRYING OUT THE INVENTION

Hereinafter, the present invention will be more specifically described by way of illustrative examples. The present invention is not so limited.

### METHODS

- Southern blot analysis
   Genomic DNA was digested with a restriction enzyme, fractioned with 0.7% agarose gel, and transferred to a Hybond-N+ nylon membrane (Amersham). A 0.7-kb EcoRI fragment of pCX-EGFP containing EGFP (Okabe M. et al. FEBS Lett 407, 313-9 (1997)) was used as a probe for detecting a transposon-specific band. Hybridization and washing were performed by standard methods (J. Sambrook et al.; Molecular Cloning: A Laboratory Manual (Cold Spring Harbor Lab. Press, Plainview, NY) (1989)). The number of copies of a transposon in a transgenic mouse was evaluated by comparing the band strength of tail DNA with that of genomic DNA derived from ES cell clones containing a single copy of the transposon using Bio imaging system BAS2500 (Fujifilm).
- PCR analysis
   An excised transposon was detected by PCR using the following primer set: TgTP-2L, 5'-ACA CAG GAA ACA GCT ATG ACC ATG ATT ACG-3', and TgTP-1U, 5'-GAC CGC TTC CTC GTG CTT TAC GGT ATC-3'. Each primer is located outside IR/DR(R) and IR/DR(L) of pTransCX-GFP:Neo. PCR was carried out using HotStarTaq system (Qiagen) under the following conditions: 95°C, 15 min, 50 cycles (94°C, 1 min; 59°C, 1 min; and 72°C, 1 min), and one cycle of 72°C, 10 min at the final process.
   The genotype of a transgenic mouse was determined using the following primer pair: for the GFP gene (EGFP-1U, 5'-CAC CCT CGT GAC CAC CCT GAC CTA-3' and EGFP-1L, 5'-CTT GAT GCC GTT CTT CTG CTT GTC G-3') and for the SB transgene (SB-2U, 5'-TCC TAG AGA TGA ACG TAC TTT GGT-3' and SB-1L, 5'-ATC CAC ATA ATT TTC CTT CCT CAT G-3'). Conditions for PCR were the same as described above, except that annealing temperature was 55°C and the number of cycles was 30. PCR products had a length of 313 bp for the GFP gene and 466 bp for the SB transgene. A flanking sequence at a novel insertion site of the transposon was PCR amplified as described above (Ivics, Z. et al., Cell 91, 501-10(1997)). The PCR products were directly sequenced using a dye terminator and the ABI373A DNA sequencer (Applied Biosystems).
- Measurement of GFP expression
   A tip of the tail of a mouse was cut off immediately after birth. The fluorescence strength of the tail tip was immediately observed using a GFP-specified filter (Olympus, Tokyo, Japan) and a fluorescence inverted microscope (Olympus) at x40 magnification. A mouse having GFP expression throughout the tail was determined to be positive.

### Example 1 Production of Plasmids and Establishment of Transgenic Mice

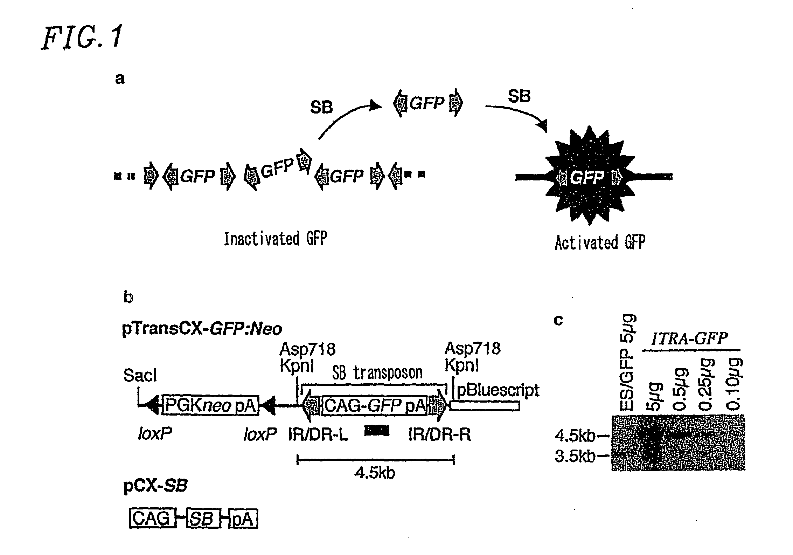

(1) Establishment of SB transgenic mice
   a) Production of a pCX-SB construct for establishment of SB transgenic mice (Figure **1b,** bottom)
      SB was excised from pSB10 plasmid (Z. Ivics et al.; Cell, 91:501-510 (1997)) using a restriction enzyme SacII, followed by blunt ending. The SB-DNA fragments were inserted into a blunt-ended EcoRI site of pCX-EGFP plasmid (M. Okabe et al.; FEBS Lett, 407:313-319 (1997)) by replacing an EGFP fragment. Thereafter, the direction of SB was determined.
   b) Establishment of SB transgenic mice from pCX-SB construct
      A unit DNA fragment containing a promoter, an SB and a poly A addition signal was excised from pCX-SB construct using a restriction enzyme SalI-BamHI, followed by purification by electrophoresis. About 5 fg of this DNA fragment was transferred into a fertilized egg of a B6C3F2 mouse, which was in turn transplanted to the oviduct of a pseudopregnant mouse. After about 20 days, a SB transgenic mouse was established (Figure **3**, upper right, "SB" mouse).
      An attempt was made to establish lines of the SB transgenic mice (Founder) by crossbreeding Founders with wild-type mice. A number of crossbreeds were required for obtaining mice containing an SB transposase gene. Only one of two Founder mice could establish a line.
(2) Establishment of transgenic mice containing a transposon sequence
   a) Production of a transposon construct pTransCX-GFP:Neo (Figure **1b,** up)
      pTransCX-GFP was produced by multiple stages. An IR/DR(R) fragment having 383 base pairs was isolated from pT/Neo plasmid (Z. Ivics et al.; Cell, 91:501-510 (1997)) by cleavage using BamHI, followedby blunt ending, and further, cleavage using EcoRI. The resultant IR/DR(R) fragment was cloned at HincII·EcoRI sites of pBluescriptII (Strategene) to obtain pBS-IR/DR(R). Similarly, an IR/DR(L) fragment having 363 base pairs was isolated from pT/Neo plasmid by cleavage using SacI, followed by blunt ending, and further, cleavage using BamHI. The resultant IR/DR(L) fragment was blunt ended at its XbaI site, followed by cloning at XbaI-BamHI sites of pBS-IR/DR(R) to obtain pBS-IR/DR(R,L). SpeI-SacI fragment of about 1.3-k base pairs containing exon 2 and intron 2 of a mouse Piga gene (K. Kawagoe et al.; Genomics, 23:566-574 (1994)) was cloned at SpeI-SacI sites of pBluescriptII to obtain pPiga. A SalI-EcoRI fragment of about 1.7-k base pairs containing a CAG promoter was excised from pCX-EGFP plasmid, followed by cloning at SalI-EcoRI sites of pPiga, to obtain pCX-Piga. An EGFP fragment was excised from pCX-EGFP plasmid using EcoRI, followed by cloning at EcoRI sites of pCX-Piga, to obtain pCX-EGFP-Piga. pCX-EGFP-Piga containing the CAG promoter was excised using SalI-SacI and its cut end was blunt ended. This fragment was blunt ended at its EcoRI and BamHI sites, followed by cloning at EcoRI-BamHI sites of pBS-IR/DR(R,L) located between IR/DR(R) and IR/DR(L), to obtain pTransCX-GFP. Finally, a 2.3-kb NotI fragment of a PGK-Neo cassette (Y. Nakano et al.; Eur. J. of Neurosci., 11:2577-2581 (1999)) interposed between two loxP sites was cloned at the unique NotI site of pTransCX-GFP to obtain pTransCX-GFP:Neo. In the drawings, a poly A signal is shortened as "pA". Transgenic mice were produced by microinjection of a long version fragment. The long version fragment was a full-length SacI fragment having the PGK-Neo cassette and the pBluescriptII vector backbone.
   b) Establishment of transposon transgenic mice from pTransCX-GFP:Neo construct
      pTransCX-GFP:Neo was linearized at the unique SacI site. About 5 fg of this DNA fragment was transferred into a fertilized egg of a B6C3F2 mouse, which was in turn transplanted to the oviduct of a pseudopregnant mouse. After about 20 days, a transposon sequence-containing mouse was established (Figure **3,** upper left, "GFP" mouse).
      The obtained transposon sequence-containing mice (Founder) were crossbred with wild-type mice at once, whereby mice which express the transposon sequence at a probability of 1/2 could be obtained. The obtained mice had the GFP gene within the transposon sequence, however, the mouse did not emit green fluorescence.
(3) Screening of transgenic mice (Figure **3)**
   a) Mice having both pTransCX-GFP:Neo and pCX-SB
      The SB transgenic mice of (1) and the transposon sequence-containing transgenic mice of (2) were crossbred. DNA was collected from the tail of resultant mice immediately after birth, followed by PCR analysis, to select transgenic mice having both pTransCX-GFP:Neo and pCX-SB ("SB-GFP" mouse in Figure **3**). The transgenic mice having both pTransCX-GFP:Neo and pCX-SB did not emit green fluorescence.
   b) Mice emitting green fluorescence, in which a transposon sequence was transposed due to a transposase
      The transgenic mice having both pTransCX-GFP:Neo and pCX-SB of a) (B line; male and female mice, A line; only male mice) and wild-type ICR mice were crossbred. A tip of the tail of the resultant mouse was cut off immediately after birth. The fluorescence strength of the tail tip was immediately observed using a GFP-specified filter (Olympus, Tokyo, Japan) and a fluorescence inverted microscope (Olympus) at x40 magnification. Mice having GFP expression and emitting green fluorescence were selected as mice in which a transposon sequence was transposed due to a transposase ("Mice with fluorescence" in Figure **3**). The results are shown in Table 1.

**[Table 1]**

| Results of Crossbreed of ITRA-GFP Double Positive Mice and Wild-type Mice | | | | | |
|---|---|---|---|---|---|
| ITRA-GFP:SB Parent mice | | Progeny | | | |
| Line | Sex | Number of all neonatal mice | GFP gene positive | GFP signal positive | Frequency of GFP active mice |
| B | Male | 61 | 36 | 23 | 63% |
| | Female | 21 | 13 | 3 | 23% |
| A | Male | 19 | 10 | 8 | 80% |

Note: The frequency of GFP active mice was calculated by dividing the number of GFP signal positive mice by the number of GFP gene positive mice. The frequency of GFP active mice indicates the rate at which the transposon was moved by the transposon system to activate the GFP gene which in turn emitted green fluorescence.

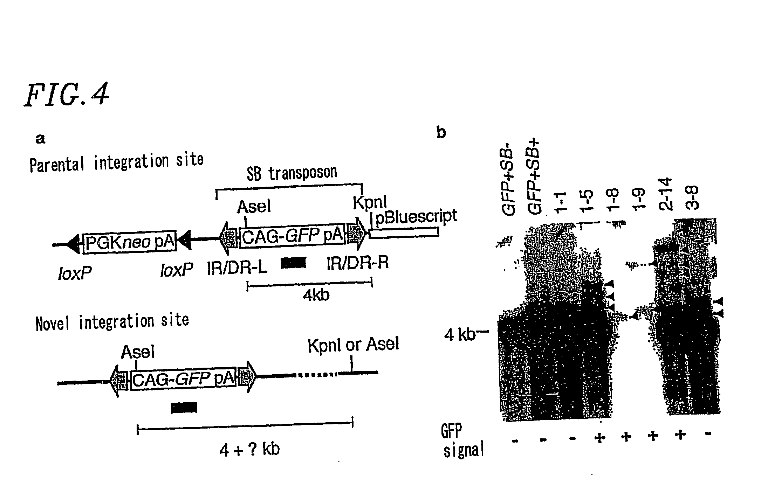

Among 36 GFP gene positive mouse individuals obtained from the B line males, 23 individuals emitted green fluorescence. Among 19 GFP gene positive mouse individuals obtained from the A line males, 10 individuals emitted green fluorescence. DNA of the obtained individuals was analyzed by southern blotting. As a result, bands which were considered to be specific to a transposition site were observed (1-5, 1-8, 1-9 and 2-14 in Figure **4b**). Further, regions surrounding the transposon sequence were amplified by PCR and analyzed. As a result, a sequence of TA-transposon sequence-TA specific to a transposition site and a genome-derived sequence at a transposition site which is not present in pTransCX-GFP:Neo were identified (1-8 and 1-9 in Figure **5**).

The presence or absence of a transposition site was investigated by southern blotting for mice which did not emit green fluorescence (the GFP gene was inactive) . As a result of this experiment, it was confirmed that whereas there were mice without a transposition site (1-1 in Figure **4b),** there were mice having a plurality of transposition sites (3-8 in Figure **4b**). Therefore, it was demonstrated that the result of Table 1 indicating the frequency of the GFP active mice was undervalued.

### INDUSTRIAL APPLICABILITY

By using an egg, a sperm, a fertilized egg, or the like derived from a transgenic non-human mammal or a founder of the present invention, lines and further established cell lines thereof can be established, which may be a useful tool for research on pathological analysis, drug screening, and the like. Specifically, it is possible to efficiently and randomly introduce mutations into genes encoding a protein involved in disease of non-human mammals. If the function of the gene can be identified, the gene and the protein encoded by the gene can be used to perform screening for a therapeutic agent capable of adjusting gene expression or protein function. If clone individuals are produced to establish a desired line, further efficient research can be expected.

From the transgenic non-human mammals of the present invention, an animal model of disease, in which a particular gene function is lost, reduced or activated so that a phenotype is changed, can be easily obtained. Such an animal model is useful as an experimental animal in the fields of medicine, pharmaceutics, and the like.

The transgenic non-human mammal of the present invention is also useful as a donor for organs, including neurons, heart, lung, liver, pancreas, kidney, cornea, skin, and the like. In a practical situation, a mammal, such as swine, which has an organ having a size similar to that of a human, is considerably useful for organ transplantation to a human.

### BRIEF DESCRIPTION OF THE DRAWINGS

Figure **1a** is a diagram for explaining a system for detecting transposition of a transposon according to one embodiment of the present invention, in which the transposon is combined with a green fluorescence protein (GFP) and a Sleeping Beauty transposase.

A GFP expression unit is shown to the left of Figure **1a,** in which a plurality of GFP genes, each of which is interposed by the terminal repeated sequences at opposite ends thereof, are linked together (the opposites ends of a "GFP" are indicated by arrows). In this situation, the GFP gene is inactivated by the sequences at the opposite ends of the transposon sequence. However, if the transposon sequence is transposed by the action of the SB transposase indicated by "SB", the sequences at the opposite ends of the transposon sequence are cut off. As a result, the GFP gene is expressed, so that green fluorescence is emitted (Figure **1a,** right)

Figure **1b** is a schematic diagram for explaining a transposon construct pTransCX-GFP:Neo (up) and a transposase construct pCX-SB (down) , which were obtained in Example 1.

In Figure **1b,** the transposon construct pTransCX-GFP:Neo contains a loxP sequence for inducing recombination of a chromosome using a Cre protein; a PGK promoter (PGK) for controlling expression of a marker gene neo; a drug resistance marker, a neomycin resistance gene (Neo), which is employed when a transposon sequence is introduced into cells; terminal repeated sequences (IR/DRs: two thick arrows pointing opposite directions) of an SB transposon containing a Direct Repeat (DR) within an Inverted Repeat (IR) ; a CAG promoter (CAG) for controlling expression of the GFP gene; a GFP gene; a Piga gene fragment-derived PolyA addition signal (pA) for stably expressing the GFP gene; and a plasmid sequence pBluescript.

The present inventors confirmed that when a short sequence of 4.5 kb obtained by digestion with KpnI (Figure **1b**) was introduced into a mouse fertilized egg, a mouse having green fluorescence was obtained no matter where the short sequence was inserted in the genome.

The transposase construct pCX-SB was an SB transposase expression vector, which contained an SB transposase gene (SB) ; a CAG promoter (CAG) for expressing the SB transposase gene; and a PolyA addition signal (pA) for stably expressing the SB transposase gene.

Figure **1c** is a diagram showing a result of analysis of the number of transposon copies by southern blotting using a GFP probe.

The result of Figure **1c** revealed that about 20 copies of pTransCX-GFP:Neo were introduced into a transgenic mouse (1TRA-GFP) obtained from a fertilized egg into which the transposon construct pTransCX-GFP:Neo had been introduced by microinjection. Note that in Figure **1c,** a 4.5-kb band was derived from pTransCX-GFP:Neo of the transgenic mouse, and a 3.5-kb band was derived from 5 µg of DNA of cells (ES/GFP) having only one copy of GFP.

Figure **2b** shows a result of PCR analysis of a transgenic mouse DNA having both or one of pTransCX-GFP:Neo and pCX-SB, indicating the presence or absence of excision of a transposon. In Figure **2b,** + and - indicate the presence (+) and absence (-) of an SB transposase gene or a GFP gene in a sample construct.

No 358-bp band was confirmed for a mouse having only pTransCX-GFP:Neo (GFP+SB-). A 358-bp DNA fragment band appeared after excision of a transposon for a mouse sample having both pTransCX-GFP:Neo and pCX-SB (GFP+SB+). Therefore, it is indicated that the excision of a transposon is mediated by the SB transposase.

Figure **2c** shows a result of investigating the frequency of excision of a transposon sequence, in which DNA derived from the tail and the blood of a sample mouse having both pTransCX-GFP:Neo and pCX-SB was repeatedly diluted by a factor of 10, followed by PCR.

Bands of Figure **2c** are specific to excision of a transposon sequence. It is suggested that excision of a transposon sequence occurred at a considerably high frequency, i.e., at least 10% of the total number of cells in the tail of the mouse sample and at least 1% in the blood.

Figure **2d** shows a signature sequence (TAcagTA or TActgTA) in which three bases were left and interposed between target sequence TAs after transposition of a transposon.

Figure **3** is schematic diagram showing an exemplary flow of producing a transgenic mouse. An example for each production method is shown in Example 1.

From the upper left of Figure **3**, "GFP" indicates a transgenic mouse having an SB transposon construct (pTransCX-GFP:Neo in Example 1); "SB" indicates a transgenic mouse having an SB transposase gene (pCX-SB in Example 1); a "SB-GFP" mouse indicates a transgenic mouse having both a transposon sequence and a transposase gene; and wt indicates a wild-type mouse. The GFP mouse and the SB-GFP mouse do not exhibit green fluorescence. A photograph is provided, which shows transgenic mice exhibiting green fluorescence in which a transposon was transposed and the GFP gene was expressed.

Figure **4a** is a restriction map of a parental integration site and a novel integration site.

A site into which a transposon construct (pTransCX-GFP:Neo) was first introduced is indicated by a parental integration site. Another site into which a transposon sequence transposed by action of a transposase was inserted is indicated by a novel integration site. As a result of digestion with AseI and KpnI, a 4-kb fragment was obtained at a first insertion site and bands having 4 kb more were obtained at subsequent insertion sites.

Figure **4b** shows a result of detection of novel insertion sites using southern blotting hybridization.

DNA of the tail of a mouse digested with AseI and KpnI was analyzed using a GFP probe. As DNA samples, a transgenic mouse having both a transposon and a transposase (GFP+SB+), a mouse having only a transposon (GFP+SB-), four mice emitting fluorescence of GFP which were progenies obtained by crossbreeding a GFP+SB+ mouse and a wild-type mouse (1-5, 1-8, 1-9 and 2-14), and two mice without fluorescence of GFP (1-1 and 3-8), were used.

The presence and absence of a GFP signal (green fluorescence) are indicated by + and -, respectively, at the bottom of the figure. Bands having more than 4 kb indicated by arrows in the figure are considered to be derived from a transposon transposition site. A strong band of 4 kb corresponds to a site into which a plurality of transposon sequences were first incorporated. It is appreciated that in all of GFP fluorescence-positive mice, a band of more than 4 kb was present, i.e., transposition occurred. It is also appreciated that since the sizes of bands differ from each other, transpositions occurred at different chromosomal sites.

Figure **5** shows base sequences of regions surrounding a parental integration site (vector) and a novel integration site (1-8 and 1-9), and base sequences of target sites of transposition. Dinucleotides TA located at a target site, which are flanking sequences at the opposite ends of a transposon sequence, are indicated by bold fonts.

Figure **6** shows a series of chromosomal deletions using an SB transposon and a Cre/loxP system according to an example of the present invention. In Figure **6,** A, B, C, D, and E represent regions on a chromosome.

A loxP sequence is introduced into each of the inside and outside of a transposon sequence (indicated by arrows pointing opposite directions). The transposon is transposed by a transposase (indicated by SB). Next, by action of a Cre recombinase (indicated by Cre) , a chromosomal region between the loxP sequence outside the transposon sequence (loxP to the left of the figure) and the loxP sequence inside the transposon (loxP to the right of the figure) can be deleted. In Figure **6,** region B is deleted in the upper sequence; regions B and C are deleted in the middle sequence; and regions B, C and D are deleted in the lower sequence.

Figure **7** shows a gene trap vector containing a splice acceptor, an IRES (internal ribosome entry site) sequence, a lacZ marker, a poly A addition signal (pA), a CAG promoter (P), a GFP marker, and a splice donor in a transposon sequence between from arrows pointing opposite directions (IR/DR-L and -R), where these elements are arranged in this order from upstream.

Figures **8a** and **8b** show a specific example of a system which adjusts ON/OFF of expression of a Bloom gene in a tetracycline-dependent manner in non-human mammals.

Figure **8a** shows a procedure in which a targeting vector is introduced into the Bloom genome so as to obtain a recombinant. A construct [(tTA-SV40poly A)-(LoxP-TKNEOpoly-LoxP)-(hGHpoly A-teto)] is introduced as a tetracycline regulatable unit into the Bloom genome, so that a recombinant Bloom genome whose transcription activity may be adjusted in a tetracycline-dependent manner is obtained.

Figure **8b** shows a mechanism by which transcription is switched ON/OFF in a tetracycline-dependent manner in the recombinant. In Figure **8b(1),** in the absence of tetracycline, a protein indicated by an ellipse is synthesized in the tetracycline regulatable unit, and this protein binds to downstream of the unit, resulting in initiation of a transcription reaction. On the other hand, in Figure **8b(2) ,** when tetracycline is added, the tetracycline inhibits the binding of the protein to downstream of the unit, resulting in inhibition of transcription of the Bloom gene.

Figure **9** shows the frequency of transposition of a transposon in a GFP-positive transgenic mouse.

For a GFP signal-positivemouse produced in an example of the present invention (a mouse without a transposase and with fixed transposition) , flanking sequences of an insertion site of a transposon sequence are identified by ligation-mediated PCR. Subsequently, nested PCR is carried out using a primer specific to the insertion site. As a result, a band specific to the insertion site is amplified (Figure **9**, up) .

When genomic DNA derived from the above-described mouse was diluted, bands were amplified for template DNA of 1-10 pg corresponding to 0.15-1.5 cells (Figure **9**, lower right). Therefore, it is considered that only one DNA molecule containing the insertion site is detectable.

DNA derived from the kidney, the spleen, the liver, the tail, and the testis of a GFP+SB+ mouse which was a parent of the above-described mouse, was used as a template for PCR. No band was detected for 0.1 µg of testis DNA. A band was amplified from 1 µg of testis DNA (Figure **9,** lower left) .

0.1 µg of DNA corresponds to 1.5×10⁴ diploid cells and 1 µg of DNA corresponds to 1.5×10⁵ diploid cells. Considering the detection sensitivity of PCR, it is considered that for the above-described mouse individual, about one per 100,000 cells contains a transposon insertion site.

For the testis, it is already known that a transposon is transposed at an average rate of about one per cell. Therefore, it is estimated that about 100,000 types of transposon insertion sites are present. In other words, it is shown that about 100,000 types of insertion sites may be found in a single individual.

## Claims

1. A transgenic non-human mammal, wherein substantially all cells thereof have at least one selected from the group consisting of at least one nonself-contained transposon and at least one signature site.

2. A transgenic non-human mammal, wherein substantially all cells thereof have (i) at least one transposase gene and at least one nonself-contained transposon or self-contained transposon and (ii) at least one signature site.

3. A transgenic non-human mammal, having a transposase gene in a state that allows the transposase gene to express a transposase.

4. A transgenic non-human mammal according to claim 1 or 2, wherein the nonself-contained transposon has at least one selected from the group consisting of at least one marker gene and at least one gene expression regulatory sequence.

5. A transgenic non-human mammal according to claim 4, wherein the marker gene is a green fluorescence protein (GFP) gene, a yellow fluorescence protein (YFP) gene, a red fluorescence protein (RFP) gene, a blue fluorescence protein (BFP) gene, a cyan fluorescence protein (CFP) gene, a lacZ gene, a luciferase gene, or a Chloramphenicol Acetyl Transferase (CAT) gene.

6. A transgenic non-human mammal according to claim 4, wherein the gene expression regulatory sequence is at least one selected from the group consisting of promoters, enhancers, insulators, silencers, splice acceptor sites, and splice donor sites.

7. A transgenic non-human mammal according to claim 2, wherein the total number of the signature sites is 0.1% or more, preferably 1% or more, and more preferably 10% or more of the total number of the cells in at least one tissue.

8. A transgenic non-human mammal according to any one of claims 1 to 3, wherein the non-human mammal is a mouse or a rat.

9. A method of producing a transgenic non-human mammal, comprising introducing a nonself-contained transposon into an animal stem cell or a fertilized egg, and obtaining a nonself-contained transposon-containing non-human mammal from the animal stem cell or the fertilized egg.

10. A method of producing a transgenic non-human mammal, comprising introducing a transposase gene into an animal stem cell or a fertilized egg, and obtaining a transposase gene-containing non-human mammal from the animal stem cell or the fertilized egg.

11. A method of producing a transgenic non-human mammal having a nonself-contained transposon and a transposase gene, comprising crossbreeding a transgenic non-human mammal containing the nonself-contained transposon and a transgenic non-human mammal containing the transposase gene.

12. A transgenic non-human mammal according to claim 2, the transgenic non-human mammal can be obtained by a method according to claim 11.

13. A method of producing a transgenic non-human mammal having a nonself-contained transposon or a signature site and having fixed transposition, comprising crossbreeding a transgenic non-human mammal having the nonself-contained transposon and the transposase gene in a state that allows the nonself-contained transposon to be transposed, and a non-human mammal containing no transposase gene.

14. A transgenic non-human mammal according to claim 1, the transgenic non-human mammal can be obtained by a method according to claim 13.

15. A method of producing a transgenic non-human mammal having a nonself-contained transposon and a transposase gene, comprising crossbreeding
(1) a nonself-contained transposon-containing non-human mammal obtained by introducing a nonself-contained transposon into an animal stem cell or a fertilized egg, and
(2) a transposase gene-containing non-human mammal obtained by introducing a transposase gene into an animal stem cell or a fertilized egg.

16. A transgenic non-human mammal according to claim 2, the transgenic non-human mammal can be obtained by a method according to claim 15.

17. A transgenic non-human mammal according to claim 16, wherein the marker gene is a green fluorescence protein (GFP) gene, a yellow fluorescence protein (YFP) gene, a red fluorescence protein (RFP) gene, a blue fluorescence gene (BFP) gene, a cyan fluorescence protein (CFP) gene, a lacZ gene, a luciferase gene, or a Chloramphenicol Acetyl Transferase (CAT) gene.

18. A transgenic non-human mammal according to claim 16, wherein the gene expression regulatory sequence is at least one selected from the group consisting of promoters, enhancers, insulators, silencers, splice acceptor sites, and splice donor sites.

19. A transgenic non-human mammal according to claim 16, wherein the total number of the signature sites is 0.1% or more, preferably 1% or more, and more preferably 10% or more of the total number of the cells in at least one tissue.

20. A transgenic non-human mammal according to claim 16, wherein the non-human mammal is a mouse or a rat.

21. A method of producing a transgenic non-human mammal having a nonself-contained transposon or a signature site and having fixed transposition, comprising
A) crossbreeding
(1) a nonself-contained transposon-containing non-human mammal obtained by introducing the nonself-contained transposon into an animal stem cell or a fertilized egg, and
(2) a transposase gene-containing non-human mammal obtained by introducing a transposase gene into an animal stem cell or a fertilized egg, and
B) crossbreeding the transgenic non-human mammal obtained in step A) having the nonself-contained transposon and the transposase gene in a state that allows the nonself-contained transposon to be transposed, and a non-human mammal containing no transposase gene.

22. A transgenic non-human mammal according to claim 1, the transgenic non-human mammal can be obtained by a method according to claim 21.

23. A transgenic non-human mammal according to claim 22, wherein the marker gene is a green fluorescence protein (GFP) gene, a yellow fluorescence protein (YFP) gene, a red fluorescence protein (RFP) gene, a blue fluorescence gene (BFP) gene, a cyan fluorescence protein (CFP) gene, a lacZ gene, a luciferase gene, or a Chloramphenicol Acetyl Transferase (CAT) gene.

24. A transgenic non-human mammal according to claim 22, wherein the gene expression regulatory sequence is at least one selected from the group consisting of promoters, enhancers, insulators, silencers, splice acceptor sites, and splice donor sites.

25. A transgenic non-human mammal according to claim 21, wherein the non-human mammal is a mouse or a rat.

26. Amethod of producing a non-human mammal model of disease, comprising selecting a non-human mammal having a disease or a pathological condition by investigating a phenotype associated with a disease of a transgenic non-human mammal according to claim 14.

27. A method of clarifying gene function, comprising investigating a change in a phenotype, and an insertion site and/or a signature site of the nonself-contained transposon or the self-contained non-human mammal, of a transgenic non-human mammal according to any one of claims 1, 2, 4-8, 12, 14, 16-20, 22-25 and 28.

28. A transgenic non-human mammal having a changed phenotype, wherein means for adjusting the efficiency of expression of a Bloom gene and a transposon system are combined, and a mutation is introduced into both alleles.
